# EUROPÄISCHE PATENTSCHRIFT

(11) **EP 1 322 583 B1**
(45) Veröffentlichungstag und Bekanntmachung des Hinweises auf die Patenterteilung: **11.01.2006**
(21) Anmeldenummer: 01972069.7
(22) Anmeldetag: 18.09.2001
(51) Int. Cl.: C07C 29/56, B01J 23/30

(54) **VERFAHREN ZUR ISOMERISIERUNG VON ALLYLALKOHOLEN**
METHOD FOR THE ISOMERISATION OF ALLYL ALCOHOLS
PROCEDE D'ISOMERISATION D'ALCOOLS ALLYLIQUES

(30) Priorität: 20.09.2000 DE 10046865
(43) Veröffentlichungstag der Anmeldung: 02.07.2003
(73) Patentinhaber: BASF Aktiengesellschaft, 67056 Ludwigshafen (DE)
(72) Erfinder: HAESE, Frank, 67245 Lambsheim (DE); EBEL, Klaus, 68623 Lampertheim (DE)
(86) Internationale Anmeldenummer: PCT/EP2001/010777
(87) Internationale Veröffentlichungsnummer: WO 2002/024617

(56) Entgegenhaltungen:
- EP-A- 0 860 415
- DE-A- 2 516 698
- PATENT ABSTRACTS OF JAPAN vol. 002, no. 034 (C-005), 8. März 1978 (1978-03-08) & JP 52 131506 A (KURARAY CO LTD), 4. November 1977 (1977-11-04)

## Beschreibung

Die vorliegende Erfindung betrifft ein Verfahren zur Isomerisierung von Edukt-Allylalkoholen zu Produkt-Allylalkoholen in beiden Richtungen des Gleichgewichtes, dadurch gekennzeichnet, dass die Umsetzung in Gegenwart eines Oxoperoxowolfram(VI)-Komplexes stattfindet sowie neue Peroxowolfram(VI)-Komplexe, sowie deren Verwendung.

Allylalkohole stellen wichtige Zwischenprodukte in der industriellen organischen Produktsynthese dar. Tertiäre Allylalkohole insbesondere dienen zum Beispiel als Zwischenverbindungen bei der Herstellung von Riechstoffen oder auch als Additive in Seifen und Detergentien.

Allylalkohole isomerisieren unter Säurekatalyse. Diese Isomerisierung entspricht einer 1,3-Wanderung der Hydroxylgruppe und einer internen Verschiebung der Doppelbindung, wie in der folgenden Gleichung mit den allgemeinen Formeln I und II dargestellt:

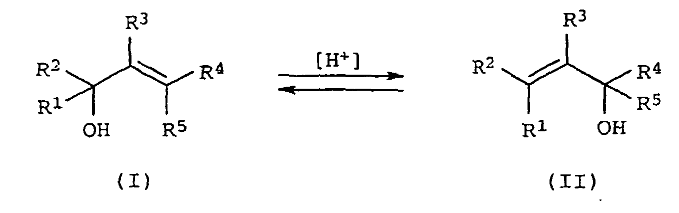

In denen die Reste R¹ bis R⁵ Wasserstoff oder einen ein oder mehrfach ungesättigten oder gesättigten C₁-C₁₂-Alkylrest bedeuten, der gegebenenfalls substituiert sein kann.

Das Verfahren ist besonders geeignet für die Herstellung von tertiären Produkt-Allylalkoholen, wie z.B. 2-Linalool, durch Isomerisieren von primären oder sekundären Allylalkoholen, wie beispielsweise Geraniol oder Nerol.

Geraniol (2-trans-3,7-Dimethyl-2,6-octadien-8-ol), Nerol (2-cis-3,7-Dimethyl-2,6-octadien-8-ol) und 2-Linalool (3,7-Dimethyl-1,6-octadien-3-ol) sind wichtige Verbindungen in der Riechstoffindustrie. Sie werden sowohl direkt als Riechstoffe eingesetzt oder durch Umsetzung mit anderen Verbindungen zu höher molekularen Riechstoffen umgesetzt. Bedeutung besitzen diese Terpenalkohole auch als C10-Bausteine in der Synthese von Vitaminen, wie dem Vitamin E und dem Vitamin A.

In der Literatur wurden in der Vergangenheit bevorzugt Verfahren zur Isomerisierung von Linalool zu Geraniol beschrieben. Da es sich bei den Isomerisierungen um Gleichgewichtsreaktionen handelt, sind die entwickelten Verfahren prinzipiell auch für die umgekehrte Reaktion der Isomerisierung von Geraniol oder Nerol zu Linalool anwendbar.

Zunächst wurden die Isomerisierungsreaktionen von Allylalkoholen mit Säuren als Katalysatoren durchgeführt. Diese Verfahren waren jedoch nur von eingeschränkter Bedeutung, da bei ihnen Nebenreaktionen, wie z.B. Dehydratisierungen oder Cyclisierungen dominierten.

Später wurden Molybdän-, Vanadium- und Wolfram-Verbindungen als Katalysatoren für die Umlagerung von substituierten Allylalkoholen identifiziert und untersucht (vgl. P. Chabardes et al. in *Tetrahedron* 33 (1977), Seiten 1775-1783.).

Während die in GB 125 6184 als Isomerisierungskatalysator beschriebene Molybdänverbindung unbefriedigende Reaktionsergebnisse ergab, waren mit Wolframoxo(VI)alkoholat-Katalysatoren der Formel WO(OR)₄ in Gegenwart einer Stickstoff-Base als zusätzlichem Liganden relativ hohe Selektivitäten bei gleichzeitig höheren Aktivitäten als mit den analogen Vanadiumoxo(V)alkoholat-Katalysatoren der Formel VO(OR)₃ möglich. Weitere Vorteile der Wolfram-Katalysatoren liegen darin, daß sie sich zum einen leicht aus dem Reaktionsgemisch abtrennen lassen (vgl. T. Hosogai et al. in *Chemistry Letters* 1982, Seiten 357-360) und daß sie im Vergleich zum Vanadiumkatalysator eine nur geringe Toxizität aufweisen.

Weiterhin ist aus DE 25 16 698 die Herstellung von neuen Katalysatoren auf der Basis von Wolfram, sowie deren Verwendung für die katalytische Umlagerung von tertiären zu primären Allylalkoholen bekannt. Als Katalysatoren werden bei diesem Verfahren Wolframoxo(VI)-Komplexe enthaltend Alkoxy-Reste und/oder über Sauerstoff gebundene Trialkylsilyl-Reste beschrieben, die zur Verbesserung der Selektivität zusätzlich koordinativ an das Wolfram gebundene Liganden enthalten, welche ein Element ausgewählt aus den Elementen N, P, As und Bi, insbesondere Liganden ausgewählt aus der Klasse der primären, sekundären und tertiären Monoamine, der Polyamine, der Schiff'schen Basen, der Imine, Nitrile und Isonitrile enthalten. Als besonders geeignete Liganden werden hierin genannt primäre Monoamine, wie Methylamin, Ethylamin, Propylamin, b-Ethoxy-ethylamin, Butylamin, Cyclohexylamin, Anilin und Naphthylamin; sekundäre Monoamine, wie Dimethylamin, Diethylamin, Dibutylamin, Dicyclohexylamin, Methylanilin, Methylcyclohexylamin, Piperidin, Morpholin und Pyrrolidin; tertiäre Monoamine, wie Trimethylamin, Triethylamin, Ethyldibutylamin, Tricyclohexylamin, Dimethylanilin, Pyridin, Picolin, Chinolin, Isochinolin, N-Methylpyrrolidin und N-Methylmorphoin; Ethylendiamin, Pyrazin, Piperazin, Pyrimidin, Triethylendiamin, 1,8-Diaza-bicyclo[5,4,0]undec-7-en, Polyethylenimine sowie Ionenaustauscherharze mit einer Vielzahl von Aminogruppen innerhalb der Moleküle, insbesondere Pyridin, Triethylamin, Cyclohexylamin, Diethylamin und Tricyclohexylphosphin. Aminoalkohole werden hierin nicht genannt.

Eigene Untersuchungen zur Isomerisierung von Geraniol mit einer 0,05 mol-%-igen Lösung von Wolframoxo(VI)-tetrakisgeranylat analog zum dem in DE 25 16 698 beschriebenen Verfahren zeigten, dass die Umlagerung zu Linalool in Gegenwart einer Stickstoff-Base bei 200°C (Reaktionszeit etwa 1 Stunde) deutlich selektiver verläuft als ohne Mitverwendung einer Stickstoff-Base. Als Stickstoffbasen wurden hierbei Diethylamin, Pyridin, Imidazol und Poly-(4-vinyl-pyridin) eingesetzt. Verbesserungen wurden durch den Zusatz von Aminoalkoholen zur Katalysatorlösung erreicht (siehe Vergleichsversuche, Beispiele 2 bis 6). Die mit diesem Verfahren erzielten Selektivitäten an Linalool sind recht gut, doch lassen die dabei erzielten Umsätze noch zu wünschen übrig.

Nachteilig wirkten sich bei diesen Versuchen also die vergleichsweise geringeren Umsätze bei gleichzeitig hohen Temperaturen von über 150°C aus, welche die Bildung von Nebenprodukten beschleunigten.

DE 25 16 698 beschreibt die Synthese von Wolframalkoholaten, durch Alkoholyse von Wolframoxotetrachlorid mit Alkoholen oder in Alkoholen gelösten Alkoholaten. Die Abtrennung des Chlorids in Form von Ammoniumchlorid mit Ammoniak oder als Natriumchlorid mit Natriummethylat ist zwar möglich aber stets unvollständig. In dem destillierten Produkt-Allylalkohol ist fast immer auch Chlor vorhanden, was dessen Qualität und Akzeptanz für Riechstoffe und Vitamine stark beeinträchtigt. Zusätzlich bewirkt das Chlorid im Wolframalkoholat selbst in ppm-Mengen nachteilig Korrosion in Metall-Kolonnen und -Reaktoren. Eine nachträgliche Abtrennung von Chlorid-Spuren über Aktivkohle-Filter oder Silber(I)oxid ist möglich, aber aufwendig und macht das Gesamtverfahren komplex und teurer.

Zudem muss Wolframoxotetrachlorid nach bekannten Methoden mit Wolframsäure und Thionylchlorid in einer vorgelagerten Reaktion erst bereitgestellt werden, so dass die Synthese von Wolframalkoholaten mit hohem Aufwand in einem mehrstufigen Verfahren erfolgen muss.

Das ebenfalls in DE 25 16 698 beschriebene Verfahren für Wolframoxo(VI)-alkoholat-Komplexe, ausgehend von Wolframtrioxid mit Hydroxyverbindungen, ist aufgrund der schlechten Ausbeuten für großtechnische Anwendungen nicht brauchbar.

Wolframalkoholate sind bekanntermaßen hydrolyseempfindlich und reagieren mit Wasser zu Alkohol und Wolframoxid. Da als Nebenreaktion der Allylalkohol-Isomerisierung stets auch Dehydratisierung erfolgt, fallen unter den Bedingungen der Allylalkohol-Isomerisierung zunehmend Wolframoxide schwerlöslich aus der Reaktionsmischung aus. Diese erhöhen die Katalysätorkosten und begünstigen weiter die Bildung von Nebenprodukten sowie Wasser aus Allylalkoholen und führen zur weiteren Verschlechterung der Selektivität.

Es war daher die Aufgabe der Erfindung, das Verfahren zur Isomerisierung von Allylalkoholen weiter so zu verbessern, dass der Wolframoxokatalysator Halogen-frei und in einfachen, kostengünstigen Schritten hergestellt werden kann, um eine Kontamination des Allylalkohols mit Halogen und darüber hinaus Korrosionsprobleme in der Anlage zu vermeiden. Weiterhin sollten durch Hydrolyse bedingte Katalysatorverluste verringert oder ganz vermieden und die Produktselektivität verbessert werden. Auch besteht weiterhin der Bedarf, die Geschwindigkeit der Gleichgewichtseinstellung zu beschleunigen und die Raumzeitausbeuten zu steigern, ohne dabei die Isomerisierungs-Temperatur weiter anheben zu müssen.

Weiterhin sollten die verbesserten Katalysatoren bei der Isomerisierung von primären oder sekundären Allylalkoholen, wie Geraniol und Nerol, zu tertiären Allylalkoholen, wie Linalool, höhere Umsätze des eingesetzten Edukt-Allylalkohols erzielen, d.h. die Geschwindigkeit der Gleichgewichtseinstellung beschleunigen. Außerdem sollten die neuen Katalysatoren einfacher herstellbar sein als die bisherigen.

Erfindungsgemäß wurde die Aufgabe mit neuen homogen gelösten Oxoperoxowolfram(VI)-Komplexen der allgemeinen Formel (III) gelöst, 5 bei gleichzeitig verbesserter Selektivität und höherer Aktivität für die Isomerisierung von Geraniol und Nerol zu Linalool.

Es können erfindungsgemäß sowohl homogene Lösungen von Oxoperoxowolfram(VI) in Wasser, Alkohol oder einem anderen Lösungsmittel verwendet werden.

Gegenstand der Erfindung ist ein Verfahren zur Isomerisierung von Edukt-Allylalkoholen der allgemeinen Formel (I) zu Produkt-Allylalkoholen der allgemeinen Formel (II) 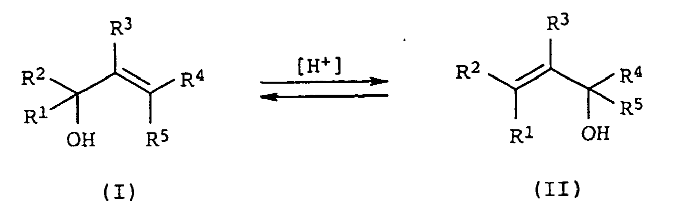
wobei R¹ bis R⁵ Wasserstoff oder einen ein oder mehrfach ungesättigten oder gesättigten C₁-C₁₂-Alkylrest bedeuten, der gegebenenfalls substituiert sein kann,
in beiden Richtungen des Gleichgewichts, dadurch gekennzeichnet, dass die Umsetzung in Gegenwart eines Oxoperoxowolfram(VI)-Komplexes der allgemeinen Formel (III) stattfindet, 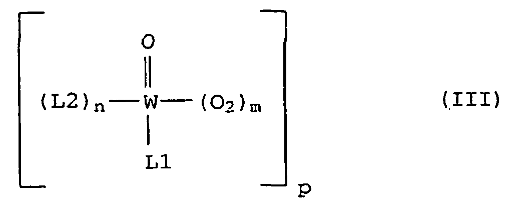
wobei
- L1, L2: jeweils unabhängig voneinander Wasser, Hydroxyl, Alkoxy oder eine freie Koordinationsstelle bedeuten oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren,
- m: die Zahl 1 oder 2 bedeutet
- n: eine Zahl von 1 bis 6 bedeutet
- p: eine Zahl zwischen 1 und 6 bedeutet, wobei im Falle von p>1 ein zwei- oder mehrkerniger Komplex gebildet wird.

Als mit Hilfe des erfindungsgemäßen Verfahrens vorteilhaft isomerisierbare Allylalkohole der allgemeinen Formel (I) oder (II) seien beispielsweise genannt: 2-Methyl-3-buten-2-ol, Prenol (3-Methyl-2-buten-1-ol), Linalool, Nerol und Geraniol sowie Farnesol (3,7,11-Trimethyl-dodeca-2,6,10-trien-1-ol) und Nerolidol (3,7,11-Trimethyl-dodeca-1,6,10-trien-3-ol), insbesondere Linalool, Nerol und Geraniol.

Die Liganden L1 und/oder L2 können sowohl Wasser, Hydroxyl, Alkoxy als auch eine freie Koordinationsstelle bedeuten oder gegebenenfalls Stickstoff oder Sauerstoff enthaltende Liganden wie Aminoalkohole, Aminophenole oder Aminocarbonsäuren oder Gemische davon bedeuten.

Als Aminoalkohole können beispielsweise verwendet werden: Triethanolamin, Diethanolamin, Monoethanolamin, Tripropanolamin, Dipropanolamin, 3-Amino-1-propanol, 1-Amino-2-propanol, 2-Amino-1-propanol, Butyldiethanolamin, Methyldiisopropanolamin, N-(3-Hydroxy-benzyl)-amin oder N,N'-Bis-(2-hydroxy-benzyl)-1,2-diaminoethan.

Als Aminophenole können beispielsweise verwendet werden: o-Aminophenol, m-Aminophenol, p-Aminophenol, oder gegebenenfalls durch Halogen, Alkyl, Amino, Hydroxy, Alkoxy, Thio, Sulfonyl, oder Nitro substituiertes 8-Hydroxychinolin, besonders bevorzugt 8-Hydroxychinolin.

Als Aminocarbonsäuren können beispielsweise verwendet werden: Picolinsäure, 2,6-Pyridindicarbonsäure oder β-Alanin.

Verwendet werden können auch Mischungen aus Aminen mit Alkoholen, Phenolen oder Carbonsäuren.

Unter den Aminen versteht man beispielsweise Mono-, Di-, oder Trimethylamin, Ethylamin, Mono-, Di- oder Triethylamin, Mono-, Di- oder Tributylamin, vorzugsweise Diethylamin.

Unter den Carbonsäuren versteht man ein oder mehrfach ungesättigte oder gesättigte Mono-, di- oder Tricarbonsäurenmit 1 bis 12 C-Atomen, die gegebenenfalls durch Hydroxy oder halogen substituiert sein können, beispielsweise Ameisensäure, Essigsäure, Buttersäure, Pivalinsäure, Buttersäure, Hexansäure, Laurinsäure, Acrylsäure, Ölsäure, Milchsäure, Weinsäure, Citronensäure, Äpfelsäure, insbesondere bevorzugt Citronensäure.

Die Phenole können gegebenenfalls durch Halogen, Alkyl, Amino, Hydroxy, Alkoxy, Thio, Sulfonyl oder Nitro substituiert sein.

Bevorzugt werden Mischungen von Diethylamin und Phenol oder Diethylamin und Citronensäure verwendet.

Unter einem Alkohol versteht man, wenn nicht anders angegeben einen Alkohol ROH, wobei R einen ein oder mehrfach ungesättigten oder gesättigten C₁-C₁₅-Alkylrest, der ein oder mehrfach durch Halogen, C₁-C₆-Alkyl, Amino oder Hydroxy substituiert sein kann, beispielsweise Methanol, Ethanol, Propanol, Isopropanol, Butanol, tert.-Butanol, Pentanol, Geraniol, Nerol, Nerolidol, Prenol, Linalool oder Farnesol.

Es können aber auch 1,3-Aminoalkohole oder 1,4-Aminoalkohole als zusätzliche Liganden verwendet werden. Die Aminoalkohole mit primärem Amin am Stickstoff-Atom können zusätzlich mono- und/oder di-alkyl-substituiert sein. Die Aminoalkohole mit einem sekundären Amin am Stickstoff-Atom können zusätzlich mono-alkyl-substituiert sein.

Bevorzugte erfindungsgemäße Katalysatoren sind solche der allgemeinen Formeln 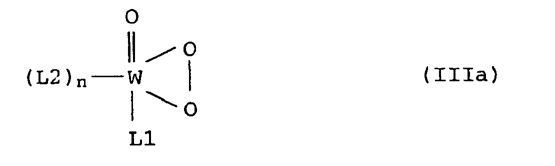 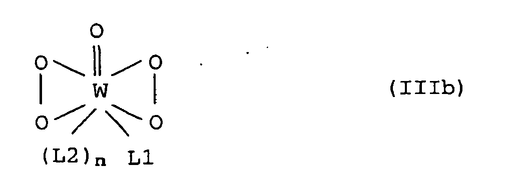 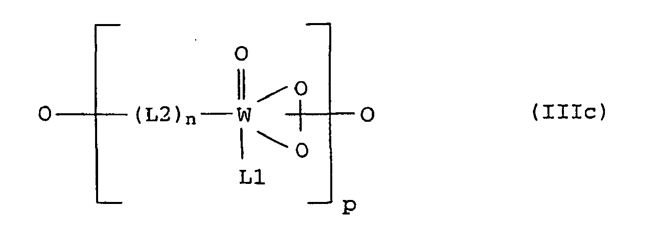
wobei L1 und L2 und n die oben angegebene Bedeutung haben und p eine Zahl von 2 bis 6 bedeutet.

Gegenstand der Erfindung sind auch neue Oxoperoxowolfram(VI)-Komplexe der allgemeinen Formel (III), 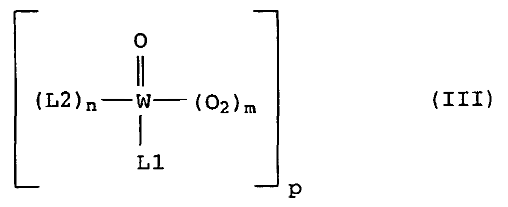
wobei
- L1: Wasser, Hydroxyl, Alkoxy oder eine freie Koordinationsstelle bedeutet oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet, und
- L2: einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet,
- m: die Zahl 1 oder 2 bedeutet
- n: eine Zahl von 1 bis 6 bedeutet
- p: eine Zahl zwischen 1 und 6 bedeutet, wobei im Falle von p>1 ein zwei- oder mehrkerniger Komplex gebildet wird.

Bevorzugt sind solche Komplexe der allgemeinen Formeln (IIIa), (IIIb) oder (IIIc), 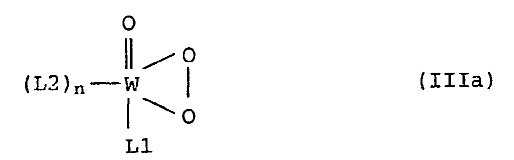 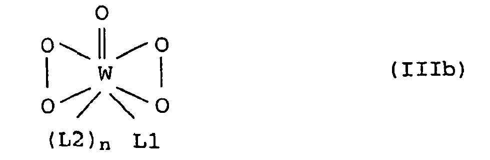 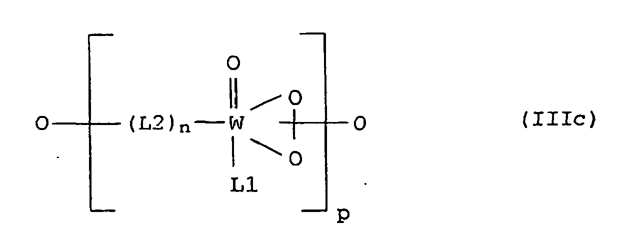
wobei
- L1: Wasser, Hydroxyl, Alkoxy oder eine freie Koordinationsstelle bedeutet oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet, und
- L2: einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet,
- n: eine Zahl von 1 bis 6 bedeutet
- p: eine Zahl zwischen 2 und 6 bedeutet, wobei im Falle von p>1 ein zwei- oder mehrkerniger Komplex gebildet wird.

Insbesondere bevorzugt sind solche Komplexe, wobei die Liganden L1 und/oder L2 8-Hydroxychinolin bedeuten.

Die erfindungsgemäßen Peroxowolfram(VI)-Komplexe können sowohl ein- als auch mehrkernig vorliegen. Liegen im Falle von p>1 die Komplexe zwei- oder mehrkernig vor, so hat der Komplex die in Formel (IIIc) angegebene Struktur.

Besonders bevorzugt wird als Ligand L1 und/oder L2 8-Hydroxychinolin eingesetzt. Das Molverhältnis Wolfram zu 8-Hydroxychinolin liegt im Bereich von 1 : 1 bis 1 : 5, vorzugsweise 1 : 1 und 1 : 2.

Die Oxoperoxowolfram(VI)-Komplexe können gemäß den in DE 195 33 331 beschriebenen Verfahren aus Wolframsäure mit wäßriger Wasserstoffperoxidlösung und gegebenenfalls anschließender Zugabe des oder der Liganden hergestellt werden.

Die Oxoperoxowolfram(VI)-Verbindungen können auch wasserfrei aus Wolframsäure und Harnstoff-Wasserstoffperoxid-Addukt und gegebenenfalls anschließender Zugabe des oder der Liganden in einem Alkohol ROH hergestellt werden, wobei die Alkohole die oben angegebene Bedeutung haben, bevorzugt Geraniol, Nerol und Linalool verwendet werden.

Der Oxoperoxowolfram(VI)-Komplex kann sowohl vor der eigentlichen Reaktion separat oder aber auch gleich in situ im Edukt-Allylalkohol hergestellt werden.

Im allgemeinen setzt man den Oxoperoxowolfram(VI)-Komplex im Edukt-Allylalkohol gelöst in einer Konzentration von 0,001 bis etwa 5 Gew.-% ein.

Die eingestellte Menge des Aminoalkohol-Liganden und insbesondere die Menge des Liganden im Verhältnis zur Menge des eingesetzten Oxoperoxowolfram(VI)-Komplexes hat Einfluß auf die Selektivität der Reaktion.

Eine niedrige Aminoalkohol-Liganden-Menge bezogen auf die Wolfram-Menge, bewirkt eine niedrigere Selektivität und Aktivität.

Als besonders vorteilhaft hat es sich erwiesen, den Liganden L1 bzw. L2 mit oder ohne ein zusätzliches organisches Lösungsmittel, zunächst mit der wäßrigen oder organischen Lösung des Oxoperoxowolfram(VI)-Komplexes zu vereinigen und erst dann die derart bereitete Lösung oder Suspension des fertigen Katalysators zum Edukt-Allylalkohol zu geben. Man verwendet den zusätzlichen Liganden im allgemeinen in einer Menge von 1 Mol-% bis 1000 Mol-%, vorzugsweise von 100 Mol-% bis 700 Mol-%, bezogen auf die Wolfram-Menge.

Die Absolutkonzentrationen von Ligand und Wolfram-Komplex in der Reaktionsmischung sind im erfindungsgemäßen Verfahren unkritisch und können beispielsweise derart erhöht werden, daß die Geschwindigkeit der Gleichgewichtseinstellung in gewünschter Weise gesteigert wird.

Eine Steigerung der Geschwindigkeit der Gleichgewichtseinstellung kann in dem erfindungsgemäßen Verfahren auch dadurch erzielt werden, daß durch Nebenreaktionen gebildetes Wasser aus der Mischung entfernt wird, beispielsweise durch Überleiten eines Inertgasstromes, Zusatz bekannter Wasser entziehender Mittel oder Strippen mit dem Produkt-Allylalkohol-Gasstrom während der Destillation.

Das erfindungsgemäße Verfahren wird im allgemeinen bei Temperaturen von 50 bis 300°C, vorzugsweise bei 150 bis 250°C, durchgeführt.

Es kann mit und ohne Verwendung eines Lösungsmittels, diskontinuierlich, aber auch kontinuierlich durchgeführt werden. Als Lösungsmittel können organische Lösungsmittel wie Toluol, Tetrahydrofuran, Benzol, Cyclohexan, Xylol, Methylenchlorid oder Mesitylen eingesetzt werden, bevorzugt aber werden die Edukt-Allylalkohole selbst als Lösungsmittel verwendet.

Das erfindungsgemäße Verfahren kann vorteilhaft durchgeführt werden, wenn man die Edukt-Allylalkohole im Reaktionsgemisch in einer Konzentration von etwa 10-Gew.-% bis zu 100 Gew.-% vorliegen hat.

Besonders vorteilhaft gestaltet sich das erfindungsgemäße Verfahren, wenn man als Edukt-Allylalkohole Geraniol und Nerol verwendet und als Produkt-Allylalkohol 2-Linalool herstellt.

Zur Aufarbeitung wird hierbei 2-Linalool als niedriger siedende Komponente durch Destillation vom Produktgemisch abgetrennt. Im allgemeinen werden Edukt-Allylalkohole und Nebenverbindungen im Produkt-Allylalkohol enthalten sein. Eine Reindestillation des Produkt-Allylalkohols kann nach bekannten Verfahren erfolgen.

Die Isomerisierung stellt eine Gleichgewichtsreaktion dar und die Gleichgewichtslage hängt von den thermodynamischen Eigenschaften der Edukt- und Produkt-Allylalkohole sowie von den Reaktionsbedingungen ab. Eine diskontinuierliche oder kontinuierliche Entfernung von Linalool, dem im Gemisch am niedrigsten siedenden Allylalkohol aus dem Reaktionsansatz erlaubt auch bei ungünstiger Gleichgewichtseinstellung aufgrund der Verschiebung des Gleichgewichtes eine günstige Raum-Zeit-Ausbeute, wobei der Sumpf der Destillationskolonne als Reaktionsraum dient.

Die folgenden Beispiele sollen das erfindungsgemäße Verfahren näher beschreiben ohne es jedoch darauf einzuschränken:

### Herstellung eines Oxoperoxowolfram(VI)-Komplexes

Beispielsweise wird Wolframsäure bei 40°C in einem 3,5-fachen Überschuss wäßrigem Wasserstoffperoxid suspendiert. Nach 6-stündigem Rühren wird die Lösung filtriert. Zu dieser bereiteten Oxoperoxowolfram(VI)-Lösung wird 8-Hydroxychinolin gegeben. Das 8-Hydroxychinolin wird als Feststoff oder als Schmelze zugesetzt oder gelöst in einem organischen Lösungsmittel, z.B. Alkohol, zugetropft. 8-Hydroxychinolin wird in einer bevorzugten Menge von 1 bis 3,5 Mol-Äquivalenten in Bezug auf das Wolfram eingesetzt. Die erhaltene Lösung oder Suspension wird direkt für die Isomerisierung mit einem Edukt-Allylalkohol eingesetzt, indem diese zu Geraniol gegeben oder Geraniol zu der Katalysator-Mischung gegeben wird. Die Komplex-Verbindung aus Wolframoxoperoxo und 8-Hydroxychinolin kann aber auch als Feststoff isoliert werden. Mischungen wäßriger oxoperoxowolfram(VI)-Lösung und alkoholischer 8-Hydroxychinolin-Lösung werden eingeengt. Das gefällte Katalysatorpulver wird abfiltriert und nach dem Waschen getrocknet. Es kann auch die nach oben beschriebener Methode bereitete Katalysator-Mischung aus Oxoperoxowolfram(VI) und 8-Hydroxychinolin nach bekannten Methoden extrahiert und das Extraktionsmittel abgezogen werden. Ebenfalls kann der Katalysator-Komplex durch die Zugabe eines Lösungsmittels, in dem sich die Komplex-Verbindung selbst schwer löst, z.B. Geraniol, gefällt werden. Es können aber auch in beliebiger Reihenfolge 8-Hydroxychinolin und Oxoperoxowolfram(VI)-Lösung getrennt nacheinander oder gleichzeitig zum Edukt-Allylalkohol gegeben werden. Der nach oben beschriebenen Verfahren vorbereitete neue Wolfram-Komplex enthält eine Peroxo-Gruppe und 8-Hydroxychinolin als weiteren Liganden und ist dadurch gekennzeichnet, dass das Verhältnis Wolfram zu 8-Hydroxychinolin 1:1 oder 1:2 beträgt.

Dieser Katalysator kann bei Temperaturen von 20 bis 300°C gelöst in einem beliebigen Lösungsmittel oder als Feststoff zum Edukt-allylalkohol gegeben werden.

Das in der Katalysator-Lösung oder -Suspension vorhandene Wasser und das organische Lösungsmittel werden vorteilhaft durch Destillation mit dem Isomerisierungsprodukt gemeinsam, z.B. mit dem tiefer siedenden Linalool, entfernt.

### Isomerisierung

Die Versuche zur Isomerisierung von Geraniol und Nerol zu 2-Linalool wurden in einem 100-ml-Dreihals-Glaskolben als Reaktionsraum mit Innenthermometer, Destillationsbrücke, Gaszuleitungsrohr und einem Magnetrührer durchgeführt. Der Reaktionskolben wurde für die Reaktion in einem Silikonölbad beheizt und es wurde ein kontinuierlicher Strom von 2 l/h Argon oder Stickstoff über die gerührte Reaktionslösung geleitet. Edukt-Allylalkohol wurde zur berechneten Katalysator-Lösung gegeben und die Mischung unter Rühren erhitzt bis die erforderliche Temperatur erreicht war. Die Versuche wurden nach 1 Stunde durch Abkühlen beendet und die Produkt-Zusammensetzung mittels GC ermittelt. Während der Reaktionen wurde kein Produkt abdestilliert.

Die Katalysatorlösung wurde hergestellt, indem 24 g (212 mmol) Wolframsäure in 30 Gew.-%iger Wassserstoffperoxid-Lösung suspendiert wurden. Die gelbe Suspension wurde 6 hr bei 40°C gerührt und nach dem Abkühlen die trübe Lösung filtriert. Diese Oxoperoxowolfram(VI)-Komplex-Lösung war gebrauchsfertig für die Isomerisierung und konnte mit der berechneten Menge eines Liganden versetzt werden. Im Falle von 8-Hydroxychinolin als Ligand wurde der Ligand als ethanolische Lösung zugegeben. Alternativ kann auch die Oxoperoxowolfram(VI)-Lösung zur Lösung des Liganden gegeben werden.

### Einfluß von Basen als Liganden

### Beispiele 1 bis 10

In einem 100-ml-Dreihals-Glaskolben wurden 120 mg (0,12 mmol W-Gehalt) der wäßrigen Oxoperoxowolfram(VI)-Lösung vorgelegt und nacheinander 0,24 mmol des aus der folgenden Tabelle 1 ersichtlichen zusätzlichen Liganden und 20 g (0,13 mol) Geraniol bei Raumtemperatur zugesetzt. Es wurde unter Rühren auf 200°C im Ölbad erwärmt und während der Reaktionszeit von 1 Stunde ein 2-1/h-Argonstrom über die Lösung geleitet. Die Reaktion wurde durch Abkühlen nach 1 h gestoppt.

Eine anschließende gaschromatographische Analyse (GC) der Reaktionsmischung ergab das aus der Tabelle 1 ersichtliche Ergebnis.

**Tabelle 1**

| Bsp | Ligand | Geraniol [GC-Fl.-%] | Nerol [GC-Fl.-%] | 2-Linalool [GC-Fl.-%] | Leichtsieder [GC-Fl.-%] | Mittel-sieder [GC-Fl.-%] | Schwersieder [GC-Fl.-%] | Selektivität zu 2-Linalool [%] |
|---|---|---|---|---|---|---|---|---|
| | | | | | | | | |
| 1 | Keiner | 94,2 | 2,60 | 1,03 | 0,81 | 0,07 | 1,28 | 32 |
| 2 | Diethylamin | 37,85 | 1,65 | 13,43 | 12,14 | 2,34 | 32,59 | 22 |
| 3 | Pyridin | 11,78 | 1,41 | 14,14 | 22,04 | 3,51 | 47,12 | 16 |
| 4 | Imidazol | 78,15 | 2,63 | 15,07 | 0,82 | 0,21 | 3,12 | 78 |
| 5 | Poly-(4-vinylpyridin) | 80,64 | 2,51 | 11,03 | 1,83 | 0,46 | 3,53 | 65 |
| 6 | 1-Amino-2-propanol | 51,44 | 4,35 | 32,58 | 3,03 | 0,81 | 7,79 | 74 |
| 7 | 8-Hydroxychinolin | 35,66 | 8,67 | 44,60 | 1,49 | 0,43 | 6,15 | 85 |
| 8 | Chinolin | 3,80 | 0,69 | 7,78 | 21,32 | 4,83 | 61,58 | 8 |
| 9 | Bispyridyl | 3,10 | 0,51 | 5,31 | 23,52 | 4,96 | 62,60 | 6 |
| 10 | o-Aminophenol | 0,22 | 0,00 | 0,97 | 28,30 | 5,96 | 64,55 | 1 |

Ohne Liganden-Zusatz zeigt der Oxoperoxowolfram(VI)-Katalysator geringe Isomerisierungsaktivität. Mit 8-Hydroxychinolin verläuft die Isomerisierung von Geraniol zu Linalool nicht nur am schnellsten sondern auch am selektivsten. In Gegenwart von Aminen allein sind Umsätze und Selektivitäten schlechter als ohne Amin-Zusatz.

Einfluß der Konzentration des Liganden am Beispiel des 8-Hydroxychinolin

### Beispiele 11 bis 17

In einem 100-ml-Dreihals-Glaskolben wurden 120 mg (0,12 mmol) der wäßrigen Oxoperoxowolfram(VI)-Lösung vorgelegt und 8-Hydroxychinolin in dem aus der folgenden Tabelle 2 ersichtlichen Molverhältnis zugegeben und 20 g (0,13 mol) Geraniol bei Raumtemperatur zugesetzt. Unter Überleiten eines kontinuierlichen Argonstroms wurde für eine Stunde auf 200°C erhitzt. Eine anschließende GC der Reaktionsmischung ergab das aus der Tabelle 2 ersichtliche Ergebnis.

**Tabelle 2**

| Bsp | Molverhältnis W:8-Hydroxychinolin | Geraniol [GC-Fl.%] | Nerol [GC-Fl.-%] | 2-Linalool [GC-Fl.-%] | Leichtsieder [GC-Fl.-%] | Mittel-sieder [GC-Fl.-%] | Schwersieder [GC-Fl.-%] | Selektivität zu 2-Linalool [%] |
|---|---|---|---|---|---|---|---|---|
| | | | | | | | | |
| 11 | 1:1 | 36,18 | 7,94 | 40,24 | 2,56 | 0,77 | 12,31 | 72 |
| 12 | 1:2 | 38,66 | 8,67 | 44,60 | 1,49 | 0,43 | 6,15 | 85 |
| 13 | 1:3 | 31,66 | 10,36 | 49,17 | 1,34 | 0,49 | 6,98 | 85 |
| 14 | 1:3,5 | 36,25 | 9,04 | 48,68 | 0,99 | 0,41 | 4,63 | 89 |
| 15 | 1:4 | 40,65 | 7,98 | 44,73 | 1,09 | 0,38 | 5,17 | 87 |
| 16 | 1:5 | 32,63 | 10,15 | 50,20 | 1,00 | 0,45 | 5,57 | 88 |
| 17 | 1:7 | 30,08 | 15,12 | 44,09 | 1,35 | 0,41 | 8,95 | 80 |

Das in Bezug auf Selektivität und Aktivität optimale Molverhältnis Wolfram:8-Hydroxychinolin liegt in einem Bereich von 1:2 bis 1:5.

### Einfluß der Temperatur

### Beispiele 18 bis 20

In einem 100-ml-Dreihals-Glaskolben wurden 120 mg (0,12 mmol) der wäßrigen Oxoperoxowolfram(VI)-Lösung vorgelegt und nacheinander 66 mg (0,455 mmol) 8-Hydroxychinolin und 20 g (0,13 mol) Geraniol bei Raumtemperatur zugesetzt. Es wurde im Ölbad im Argonstrom unter Rühren auf die in der Tabelle 3 ersichtliche Temperatur erwärmt. Eine anschließende GC der Reaktionsmischung ergab das aus der Tabelle 3 erkennbare Ergebnis.

**Tabelle 3**

| Bsp | Temperatur (°C) | Geraniol [GC-Fl.-%] | 2-Linalool [GC-Fl.-%] | Nerol [GC-Fl.-%] | Leichtsieder [GC-Fl.-%] | Mittelsieder [GC-Fl.-%] | Schwersieder [GC-Fl.-%] | Selektivität zu 2-Linalool [%] |
|---|---|---|---|---|---|---|---|---|
| | | | | | | | | |
| 18 | 170 | 59,65 | 32,16 | 4,05 | 0,78 | 0,2 | 3,16 | 89 |
| 19 | 180 | 56,89 | 34,79 | 4,73 | 0,57 | 0,19 | 2,85 | 91 |
| 20 | 190 | 34,10 | 49,21 | 8,69 | 1,28 | 0,42 | 6,03 | 86 |

Bei Temperaturen tiefer als 200°C nehmen mit 8-Hydroxychinolin die Reaktionsgeschwindigkeiten mit der Temperatur ab. Die höchste Selektivität für die Isomerisierung wurde bei einer Temperatur von 180°C gefunden. Dabei verlief die Isomerisierung mit noch guter Geschwindigkeit.

### Einfluss der Katalysatorvorbereitung

### Beispiel 21 und Beispiel 7 (Vergleichsbeispiel)

Der Katalysator Oxoperoxowolfram(VI) wurde in einer wasserfreien alkoholischen Lösung hergestellt, indem Wolframsäure in einem 3,3-fachen molaren Überschuss einer ethanolischen Lösung von Harnstoff-Wasserstoffperoxid-Addukt bei 40°C 20 Stunden gerührt wurde. Die Bestimmung des Wolframgehaltes der filtrierten homogenen Lösung ergab 90 % des theoretischen Wertes Wolfram. Analog zu Beispiel 7 wurden 0,12 mmol der Katalysatorlösung vorgelegt und nacheinander ein 3,5-facher molarer Überschuss 8-Hydroxychinolin und 20 Geraniol zugegeben. Der Reaktionskolben wurde im Argonstrom eine Stunde auf 200°C aufgeheizt.

**Tabelle 4**

| Bsp | Lösungsmittel für Katalysator | Geraniol [GC-Fl.-%] | Nerol [GC-Fl.-%] | 2-Linalool [GC-Fl.-%] | Leicht-sieder [GC-Fl.-%] | Mittelsieder [GC-Fl.-%] | Schwersieder [GC-Fl.-%] | Selektivität zu 2-Linalool [%] |
|---|---|---|---|---|---|---|---|---|
| | | | | | | | | |
| 7 | H₂O₂ in Wasser | 38,66 | 8,67 | 44,60 | 1,49 | 0,43 | 6,15 | 85 |
| 21 | HamstoffxH₂O₂ in EtOH | 40,76 | 7,66 | 45,29 | 0,78 | 0,36 | 5,16 | 88 |

Der wasserfrei in Ethanol aus Wolframsäure und Harnstoff-Wasserstoffperoxid-Addukt hergestellte Oxoperoxowolfram(VI)-Katalysator zeigt unter den Bedingungen der Isomerisierung ähnlich gute Aktivität und Selektivität wie der in wäßrigem Wasserstoffperoxid hergestellte Katalysator.

### Einfluss des Edukt-Allylalkohols

### Beispiel 22 und Beispiel 14

Analog zu Beispiel 14 wurde die Reaktion mit isomerenreinem Nerol durchgeführt. Reaktionsbedingungen sowie Edukt- und Katalysatorzusammensetzungen waren mit Beispiel 14 identisch.

**Tabelle 5**

| Bsp | Molverhältnis W:8-Hydroxychinolin | Geraniol [GC-Fl.-%] | Nerol [GC-Fl.-%] | 2-Linalool [GC-Fl.-%] | Leichtsieder [GC-Fl.-%] | Mittelsieder [GC-Fl.-%] | Schwersieder [GC-Fl.-%] | Selektivität zu 2-Linalool [%] |
|---|---|---|---|---|---|---|---|---|
| | | | | | | | | |
| 14 | 1:3,5 | 36,25 | 9,04 | 48,68 | 0,99 | 0,41 | 4,63 | 89 |
| 22 | 1:3,5 | 11,82 | 32,97 | 44,14 | 3,37 | 0,85 | 6,85 | 80 |

Die Isomerisierung von Nerol zu Linalool verläuft ähnlich gut wie mit Geraniol. Während der Isomerisierung wird durch Einstellung des Gleichgewichtes über Linalool aus Nerol auch Geraniol und entsprechend aus Geraniol auch Nerol gebildet.

Synthese der Oxoperoxowolfram-Katalysatoren und zu deren Verwendung in der Isomerisierung von Geraniol und Nerol:

### Synthese:

### Beispiel 23

Zu einer Lösung von 9,896 g 8-Hydroxychinolin in 160 ml Ethanol werden bei Raumtemperatur und unter Rühren 19,5 g einer wäßrigen Oxoperoxowolfram(VI)-Lösung (Wolfram-Gehalt: 19 Gewichts-%) gegeben. Die klare Lösung färbt sich augenblicklich intensiv gelb und nach der weiteren Zugabe von 90 ml Ethanol fallen innerhalb von wenigen Stunden etwa 10 g eines gelben Kristallpulvers aus. Die Kristalle werden mit wenig Ethanol gewaschen und an der Luft getrocknet.

Mikroanalyse: C: 39,1 %; O: 19,3 %; N: 5,0 %; H: 2,7 %; W: 30,6 %

### Beispiel 24

1,2 g einer wäßrigen Oxoperoxowolfram(VI)-Lösung (enthält 1,2 mmol Wolfram) werden nacheinander mit 10 ml Ethanol und 190 mg (1,31 mmol) 8-Hydroxychinolin in 3 ml Ethanol versetzt. Die erst farblose Lösung färbt sich momentan intensiv gelb und bleibt klar. Diese gelbe Lösung wird bei Raumtemperatur mit 70 ml Geraniol oder 70 ml Nerol oder einer Mischung aus Geraniol und Nerol versetzt und gerührt. Die Lösung geht innerhalb von Minuten von gelb über orange nach rotbraun über und es bilden sich 126 mg einer rotbraunen, feinkristallinen Fällung.

Mikroanalyse: C: 30,7 %; O: 16 %; N: 3,9%; H: 2,2 %; W: 46 %

### Katalyse:

### Beispiel 25

In einem 500-ml-Rührkolben mit Strömungsbrecher, Blattrührer und Destillationsbrücke wurden 250 g einer Geraniol/Nerol-Mischung im Verhältnis 2:1 vorgelegt und im Argonstrom (1,3 l/h) auf 180°C aufgeheizt. In der Hitze wurden 900 mg des Katalysator-Pulvers aus Beispiel 23 zugegeben und für eine Stunde bei 180°C gerührt. Eine anschließende GC der Reaktionsmischung ergab das aus der Tabelle 6 erkennbare Ergebnis.

### Beispiel 26

In einem 100-ml-Dreihals-Glaskolben wurden 17,7 mg des Katalysator-Pulvers aus Beispiel 24 vorgelegt und 7 g isomerenreines Geraniol zugegeben. Es wurde im Ölbad im Argonstrom unter Rühren auf 180°C erwärmt. Nach einer Stunde ergab eine anschließende GC-Analyse der Reaktionsmischung das aus Tabelle 6 ersichtliche Ergebnis.

**Tabelle 6**

| Bsp | Katalysator aus Beispiel | Geraniol [GC-Fl.-%] | Nerol [GC-Fl.-%] | 2-Linalool [GC-Fl.-%] | Leichtsieder [GC-Fl.-%] | Mittelsieder [GC-Fl.-%] | Schwersieder [GC-Fl.-%] | Selektivität zu 2-Linalool [%] |
|---|---|---|---|---|---|---|---|---|
| | | | | | | | | |
| 25 | 23 | 36,16 | 17,03 | 42,18 | 1,48 | 0,25 | 3,03 | 90 |
| 26 | 24 | 47,74 | 5,42 | 42,54 | 0,94 | 0,15 | 3,22 | 91 |

In Abhängigkeit vom Überschuss 8-Hydroxychinolin, in Bezug auf Wolfram, bildeten sich unterschiedliche Oxoperoxowolfram-Komplexe, welche durch Elementaranalyse und IR-Spektroskopie charakterisiert wurden. Beide Katalysator-Spezies sind katalytisch aktiv mit vergleichbaren Selektivitäten in Bezug auf die Bildung von Linalool aus Geraniol und Nerol.

### Allgemeine Erläuterung der Ergebnisse:

Nach Beendigung der Reaktion durch Abkühlen wurde die Produkt-Zusammensetzung mittels GC bestimmt. Bei den Leichtsiedern handelt es sich um Dehydratisierungsprodukte aus Geraniol, Nerol und Linalool. Schwersieder entstanden durch Etherbildung aus Geraniol, Nerol und Linalool. Die Selektivität für Linalool wurde ermittelt aus dem Quotienten des Umsatzes zu Linalool und der Summe aus den Produkten Linalool, Leicht-, Mittel- und Hochsieder.

## Patentansprüche

1. Verfahren zur Isomerisierung von Edukt-Allylalkoholen der Formel (I) zu Produkt-Allylalkoholen der allgemeinen Formel (II) 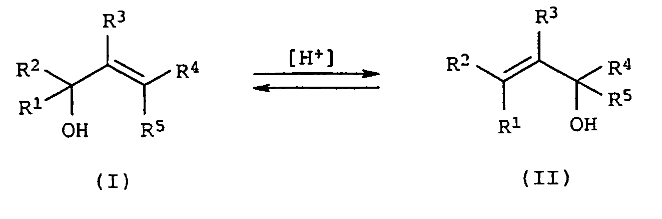
wobei R¹ bis R⁵ Wasserstoff oder einen ein oder mehrfach ungesättigten oder gesättigten C₁-C₁₂-Alkylrest bedeuten, der gegebenenfalls substituiert sein kann,
in beiden Richtungen des Gleichgewichts, **dadurch gekennzeichnet, dass** die Umsetzung in Gegenwart eines Oxoperoxowolfram(VI)-Komplexes der allgemeinen Formel (III) stattfindet, 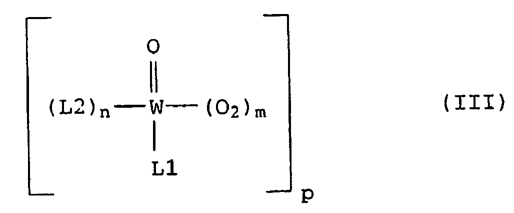
wobei
L1, L2 jeweils unabhängig voneinander Wasser, Hydroxy, Alkoxy oder eine freie Koordinationsstelle bedeuten oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren,
m die Zahl 1 oder 2 bedeutet
n eine Zahl von 1 bis 6 bedeutet
p eine Zahl von 1 bis 6 bedeutet, wobei im Falle von p>1 ein zwei- oder mehrkerniger Komplex gebildet wird.

2. Verfahren gemäß Anspruch 1, **dadurch gekennzeichnet, dass** der Komplex der allgemeinen Formel (III), ausgewählt ist aus der Gruppe der allgemeinen Formeln (IIIa), (IIIb) oder (IIIc) 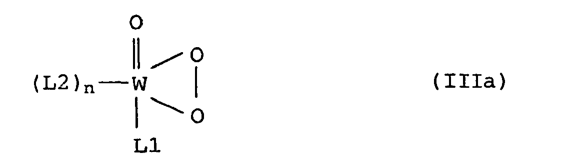 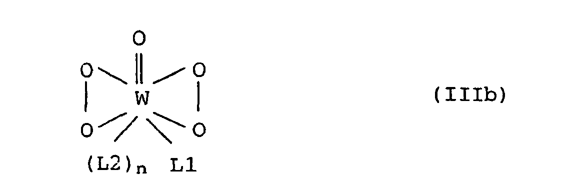 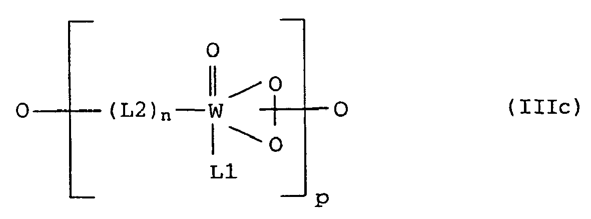
wobei L1, L2 und n die in Anspruch 1 angegebene Bedeutung haben und p eine Zahl von 2 bis 6 bedeutet.

3. Verfahren gemäß einem der Ansprüche 1 oder 2, **dadurch gekennzeichnet, dass** L1 und/oder L2 ausgewählt sind aus der Gruppe Triethanolamin, Diethanolamin, Monoethanolamin, Tripropanolamin, Dipropanolamin, 3-Amino-1-propanol, 1-Amino-2-propanol, 2-Amino-1-propanol, Butyldiethanolamin, Methyldiisopropanolamin, N-(2-Hydroxy-benzyl)-aminooder N,N'-Bis-(2-hydroxybenzyl)-1,2-diaminoethan.

4. Verfahren gemäß einem der Ansprüche 1 bis 3, **dadurch gekennzeichnet, dass** L1 und/oder L2 ausgewählt sind aus der Gruppe o-Aminophenol, m-Aminophenol, p-Aminophenol, oder gegebenenfalls durch Halogen, Alkyl, Amino, Hydroxy, Alkoxy, Thio, Sulfonyl oder Nitro substituiertes 8-Hydrok-ychinolin, Picolinsäure, 2,6-Pyridindicarbonsäure oder β-Alanin.

5. Verfahren gemäß einem der Ansprüche 1 bis 4, **dadurch gekennzeichnet, dass** L1 und/oder L2 Gemische von Diethylamin und Phenol oder Diethylamin und Citronensäure bedeuten.

6. Verfahren gemäß einem der Ansprüche 1 bis 5, **dadurch gekennzeichnet, dass** L1 und/oder L2 8-Hydroxychinolin bedeutet.

7. Verfahren gemäß einem der Ansprüche 1 bis 6, **dadurch gekennzeichnet, dass** das Verhältnis Wolfram zu 8-Hydroxychinolin zwischen 1 : 1 und 1 : 5 liegt.

8. Verfahren gemäß einem der Ansprüche 1 bis 7, **dadurch gekennzeichnet, dass** als Edukt Allylalkohol primäre oder sekundäre Allylalkohole eingesetzt werden.

9. Verfahren gemäß einem der Ansprüche 1 bis 8, **dadurch gekennzeichnet, dass** die Allylalkohole ausgewählt sind aus der Gruppe 2-Methyl-3-buten-2-ol, Prenol (3-Methyl-2-buten-1-ol), Linalool, Nerol, Geraniol, Farnesol (3,7,11-Trimethyl-dodeca-2,6,10-trien-1-ol) oder Nerolidol (3,7,11-Trimethyl-dodeca-1,6,10-trien-3-ol).

10. Verfahren gemäß einem der Ansprüche 1 bis 9, **dadurch gekennzeichnet, dass** die Edukt-Allylalkohole ausgewählt sind aus der Gruppe Geraniol oder Nerol.

11. Verfahren gemäß einem der Ansprüche 1 bis 10, **dadurch gekennzeichnet, dass** der Oxoperoxowolfram(VI)-Komplex der allgemeinen Formel (III) vor der Reaktion oder in situ im Edukt-Allylalkohol hergestellt wird.

12. Oxoperoxowolfram(VI)-Komplexe der allgemeinen Formel (III), 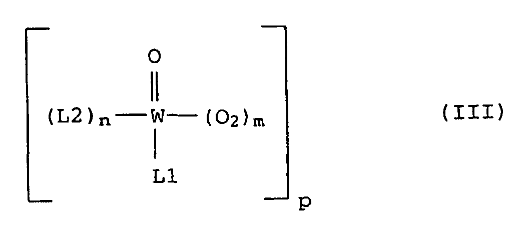
wobei
L1 Wasser, Hydroxyl, Alkoxy oder eine freie Koordinationsstelle bedeutet oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet, und
L2 einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet,
m die Zahl 1 oder 2 bedeutet
n eine Zahl von 1 bis 6 bedeutet
p eine Zahl zwischen 1 und 6 bedeutet.

13. Oxoperoxowolfram(VI)-Komplexe gemäß Anspruch 12, **dadurch gekennzeichnet, daß** die Komplexe ausgewählt sind aus den allgemeinen Formeln (IIIa), (IIIb) oder (IIIc), 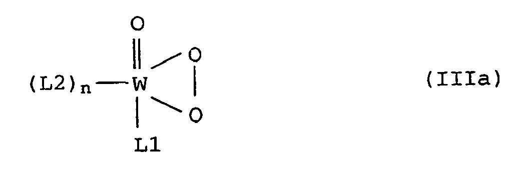 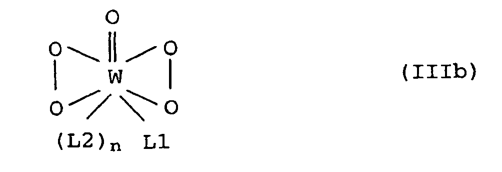 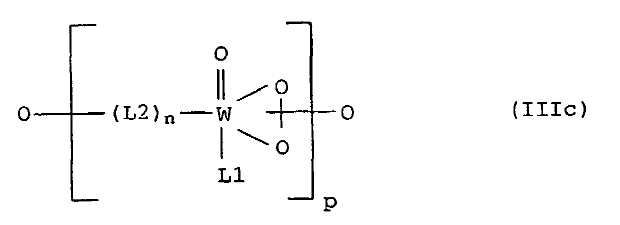
wobei
L1 Wasser, Hydroxyl, Alkoxy oder eine freie Koordinationsstelle bedeutet oder gegebenenfalls einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet, und
L2 einen Liganden ausgewählt aus der Gruppe der Aminoalkohole, der Aminophenole, der Aminocarbonsäuren oder Gemischen davon oder Gemischen von Aminen mit Alkoholen, Phenolen oder Carbonsäuren bedeutet,
n eine Zahl von 1 bis 6 bedeutet
p eine Zahl zwischen 2 und 6 bedeutet.

14. Oxoperoxowolfram(VI)-Komplexe gemäß einem der Ansprüche 12 oder 13, **dadurch gekennzeichnet, dass** L1 und/oder L2 8-Hydroxychinolin bedeuten.

15. Verwendung der Verbindungen der allgemeinen Formel (III) als Katalysatoren bei der Isomerisierung von Allylalkoholen.

## Claims

1. A process for the isomerization of precursor allyl alcohols of the formula (I) to product allyl alcohols of the formula (II) 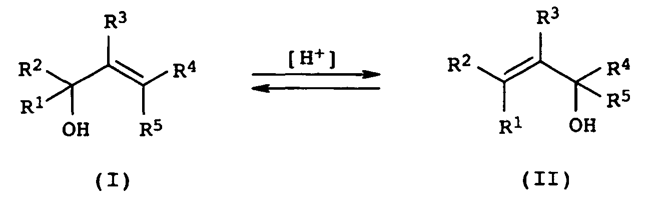
where R¹ to R⁵ are hydrogen or a mono- or polyunsaturated or saturated C₁-C₁₂-alkyl radical, which may be optionally substituted,
in both directions of the equilibrium, wherein the reaction takes place in the presence of an oxoperoxotungsten(VI) complex of the formula (III), 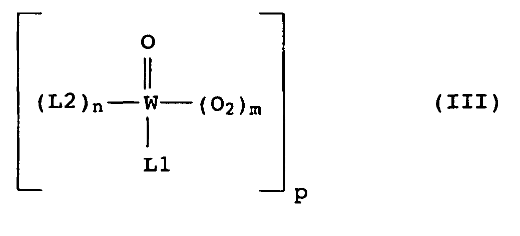
where
L1, L2 in each case independently of one another are water, hydroxyl, alkoxy or a free coordination site, or if appropriate are a ligand chosen from the group of amino alcohols, aminophenols, aminocarboxylic acids or mixtures thereof or mixtures of amines with alcohols, phenols or carboxylic acids,
m is the number 1 or 2
n is a number from 1 to 6
p is a number from 1 to 6, where in the case of p>1 a bi- or polynuclear complex is formed.

2. The process according to Claim 1, wherein the complex of the formula (III) is chosen from the group of the formulae (IIIa), (IIIb) and (IIIc) 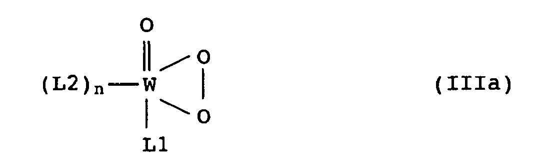 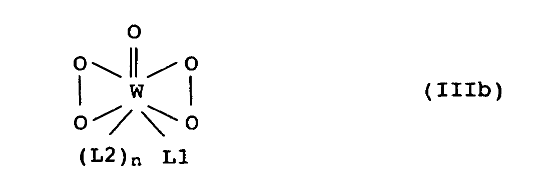 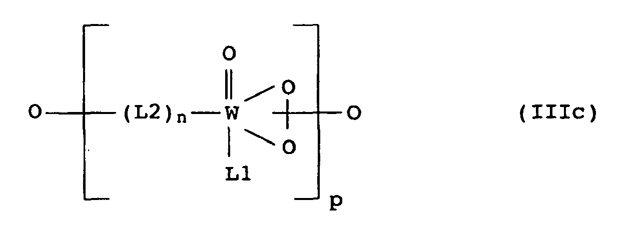
where L1, L2 and n are as defined in claim 1, and p is a number from 2 to 6.

3. The process according to either of claims 1 and 2, wherein L1 and/or L2 are chosen from the group triethanolamine, diethanolamine, monoethanolamine, tripropanolamine, dipropanolamine, 3-amino-1-propanol, 1-amino-2-propanol, 2-amino-1-propanol, butyldiethanolamine, methyldiisopropanolamine, N-(2-hydroxybenzyl)amine and N,N'-bis(2-hydroxybenzyl)-1,2-diaminoethane.

4. The process according to any of claims 1 to 3, wherein L1 and/or L2 are chosen from the group o-aminophenol, m-aminophenol, p-aminophenol, 8-hydroxyquinoline optionally substituted by halogen, alkyl, amino, hydroxyl, alkoxy, thio, sulfonyl or nitro, picolinic acid, 2,6-pyridinedicarboxylic acid and β-alanine.

5. The process according to any of claims 1 to 4, wherein L1 and/or L2 are mixtures of diethylamine and phenol or diethylamine and citric acid.

6. The process according to any of claims 1 to 5, wherein L1 and/or L2 are 8-hydroxyquinoline.

7. The process according to any of claims 1 to 6, wherein the ratio of tungsten to 8-hydroxyquinoline is between 1 : 1 and 1 : 5.

8. The process according to any of claims 1 to 7, wherein primary or secondary allyl alcohols are used as precursor allyl alcohol.

9. The process according to any of claims 1 to 8, wherein the allyl alcohols are chosen from the group 2-methyl-3-buten-2-ol, prenol (3-methyl-2-buten-1-ol), linalool, nerol, geraniol, farnesol (3,7,11-trimethyldodeca-2,6,10-trien-1-ol) and nerolidol (3,7,11-trimethyldodeca-1,6,10-trien-3-ol).

10. The process according to any of claims 1 to 9, wherein the precursor allyl alcohols are chosen from the group geraniol and nerol.

11. The process according to any of claims 1 to 10, wherein the oxoperoxotungsten(VI) complex of the formula (III) is prepared prior to the reaction or in situ in the precursor allyl alcohol.

12. An oxoperoxotungsten(VI) complex of the formula (III), 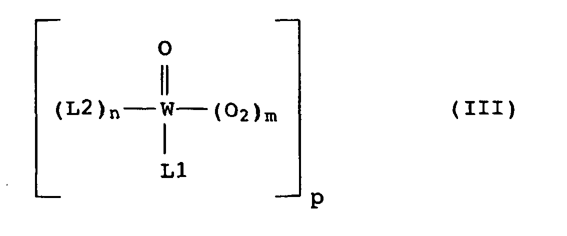
where
L1 is water, hydroxyl, alkoxy or a free coordination site, or if appropriate is a ligand chosen from the group of amino alcohols, aminophenols, aminocarboxylic acids or mixtures thereof or mixtures of amines with alcohols, phenols or carboxylic acids, and
L2 is a ligand chosen from the group of amino alcohols, aminophenols, aminocarboxylic acids or mixtures thereof or mixtures of amines with alcohols, phenols or carboxylic acids,
m is the number 1 or 2
n is a number from 1 to 6
p is a number between 1 and 6.

13. The oxoperoxotungsten(VI) complex according to claim 12, which is chosen from the formulae (IIIa), (IIIb) and (IIIc), 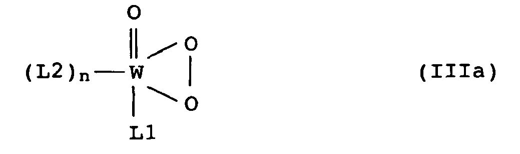 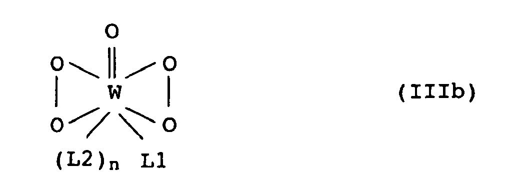 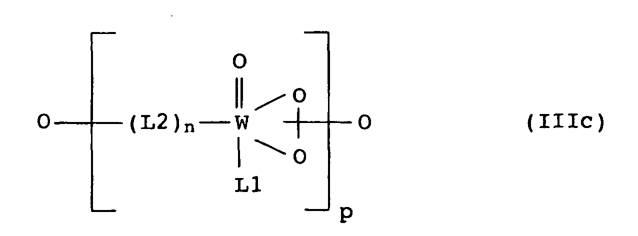
where
L1 is water, hydroxyl, alkoxy or a free coordination site, or if appropriate is a ligand chosen from the group of amino alcohols, aminophenols, aminocarboxylic acids or mixtures thereof or mixtures of amines with alcohols, phenols or carboxylic acids, and
L2 is a ligand chosen from the group of amino alcohols, aminophenols, aminocarboxylic acids or mixtures thereof or mixtures of amines with alcohols, phenols or carboxylic acids,
n is a number from 1 to 6
p is a number between 2 and 6.

14. The oxoperoxotungsten(VI) complex according to either of claims 12 and 13, wherein L1 and/or L2 are 8-hydroxyquinoline.

15. The use of compounds of the formula (III) as catalysts in the isomerization of allyl alcohols.

## Revendications

1. Procédé d'isomérisation d'alcools allyliques en éduit de formule (I) en alcools allyliques en produit de formule générale (II) 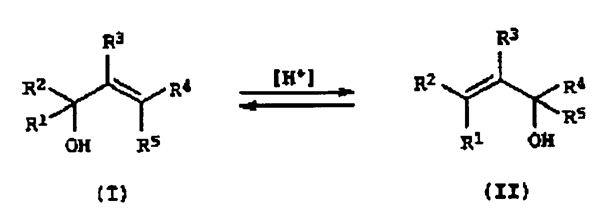
dans laquelle R¹ à R⁵ désignent l'hydrogène ou un reste alkyle en C₁ à C₁₂ mono- ou polyinsaturé ou saturé qui peut éventuellement être substitué, dans les deux directions de l'équilibre, **caractérisé en ce que** la mise en réaction se déroule en présence d'un complexe d'oxoperoxotungstène (VI) de formule générale (III) 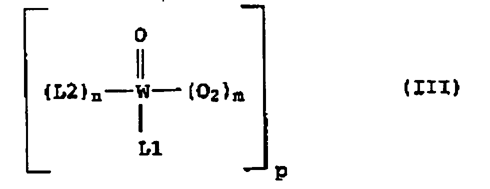
dans laquelle
L1, L2 désignent respectivement, indépendamment l'un de l'autre, l'eau, un radical hydroxy ou alcoxy ou une position de coordination libre ou, éventuellement, un ligand sélectionné dans le groupe des aminoalcools, des aminophénols, des acides aminocarboxyliques ou des mélanges de ceux-ci ou des mélanges d'amines avec des alcools, des phénols ou des acides carboxyliques,
m désigne le nombre 1 ou 2
n désigne un nombre de 1 à 6
p désigne un nombre de 1 à 6, un complexe bivalent
ou polyvalent étant formé dans le cas de p>1.

2. Procédé selon la revendication 1, **caractérisé en ce que** le complexe de formule générale (III) est sélectionné à partir du groupe des formules générales (IIIa), (IIIb), (IIIc) 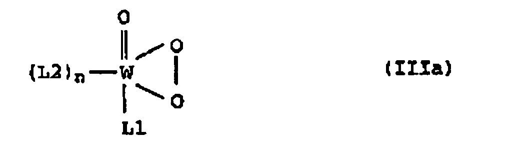 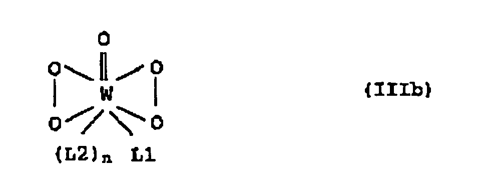 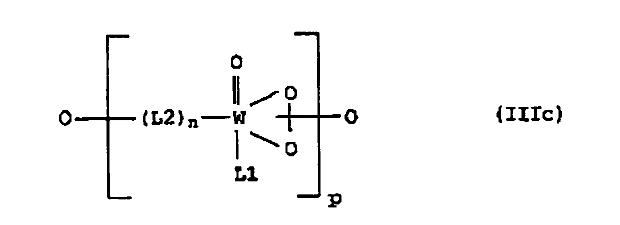
dans lesquelles L1, L2 et n ont la signification indiquée dans la revendication 1 et p désigne un nombre de 2 à 6.

3. Procédé selon l'une des revendications 1 ou 2, **caractérisé en ce que** L1 et/ou L2 sont sélectionnés dans le groupe composé de la triéthanolamine, de la diéthanolamine, de la monoéthanolamine, de la tripropanolamine, de la dipropanolamine, du 3-amino-1-propanol, du 1-amino-2-propanol, du 2-amino-1-propanol, de la butyldiéthanolamine, de la méthyldiisopropanolamine, de la N-(2-hydroxybenzyl)-amine ou du N,N'-bis-(2-hydroxybenzyl)-1,2-diaminoéthane.

4. Procédé selon l'une des revendications 1 à 3, **caractérisé en ce que** L1 et/ou L2 sont sélectionnés dans le groupe o-aminophénol, m-aminophénol, p-aminophénol ou de 8-hydroxyquinoléine, substituée éventuellement par un halogène, un radical alkyle, amino, hydroxy, alcoxy, thio, sulfonyle ou nitro, l'acide picolinique, l'acide 2,6-pyridinedicarboxylique ou la β-alanine.

5. Procédé selon l'une des revendications 1 à 4, **caractérisé en ce que** L1 et/ou L2 désignent des mélanges de diéthylamine et de phénol ou de diéthylamine et d'acide citrique.

6. Procédé selon l'une des revendications 1 à 5, **caractérisé en ce que** L1 et/ou L2 désignent la 8-hydroxyquinoléine.

7. Procédé selon l'une des revendications 1 à 6, **caractérisé en ce que** le rapport du tungstène à la 8-hydroxyquinoléine se situe entre 1:1 et 1:5.

8. Procédé selon l'une des revendications 1 à 7, **caractérisé en ce que** des alcools allyliques primaires ou secondaires sont utilisés comme alcool allylique en éduit.

9. Procédé selon l'une des revendications 1 à 8, **caractérisé en ce que** les alcools allyliques sont sélectionnés dans le groupe 2-méthyl-3-butén-2-ol, le prénol (3-méthyl-2-butén-1-ol), le linalool, le nérol, le géraniol, le farnésol (3,7,11-triméthyl-dodéca-2,6,10-trién-1-ol) ou le nérolidol (3,7,11-triméthyl-dodéca-1,6,10-trién-3-ol).

10. Procédé selon l'une des revendications 1 à 9, **caractérisé en ce que** les alcools allyliques en éduit sont sélectionnés dans le groupe géraniol ou nérol.

11. Procédé selon l'une des revendications 1 à 10, **caractérisé en ce que** le complexe d'oxoperoxotungstène (VI) de formule générale (III) est préparé avant la réaction ou in-situ dans l'alcool allylique en éduit.

12. Complexes d'oxoperoxotungstène (VI) de formule générale (III) 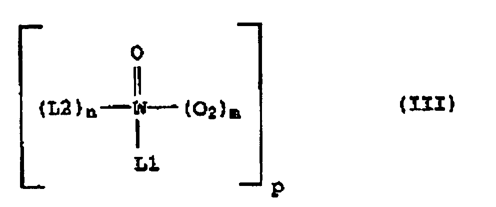
dans laquelle
L1 désigne l'eau, un radical hydroxyle, alcoxy ou une position de coordination libre ou, éventuellement, un ligand sélectionné dans le groupe des aminoalcools, des aminophénols, des acides aminocarboxyliques ou des mélanges de ceux-ci ou des mélanges d'amines avec des alcools, des phénols ou des acides carboxyliques et
L2 désigne un ligand sélectionné dans le groupe des aminoalcools, des aminophénols, des acides aminocarboxyliques ou des mélanges de ceux-ci ou des mélanges d'amines avec des alcools, des phénols ou des acides carboxyliques,
m désigne le nombre 1 ou 2
n désigne un nombre de 1 à 6
p désigne un nombre entre 1 et 6.

13. Complexes d'oxoperoxotungstène (VI) selon la revendication 12, **caractérisés en ce que** les complexes sont sélectionnés à partir du groupe des formules générales (IIIa), (IIIb) ou (IIIc) 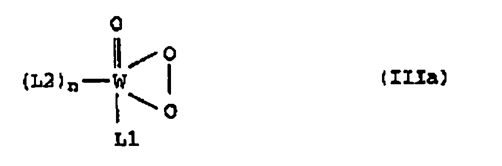 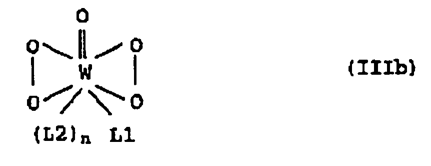 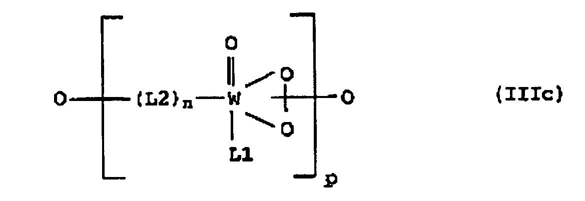
dans lesquelles
L1 désigne l'eau, un radical hydroxyle, alcoxy ou une position de coordination libre ou, éventuellement, un ligand sélectionné dans le groupe des aminoalcools, des aminophénols, des acides aminocarboxyliques ou des mélanges de ceux-ci ou des mélanges d'amines avec des alcools, des phénols ou des acides carboxyliques et
L2 désigne un ligand sélectionné dans le groupe des aminoalcools, des aminophénols, des acides aminocarboxyliques ou des mélanges de ceux-ci ou des mélanges d'amines avec des alcools, des phénols ou des acides carboxyliques,
n désigne un nombre de 1 à 6
p désigne un nombre entre 2 et 6.

14. Complexes d'oxoperoxotungstène (VI) selon l'une des revendications 12 ou 13, **caractérisés en ce que** L1 et/ou L2 désignent la 8-hydroxyquinoléine.

15. Mise en oeuvre des composés de formule générale (III) comme catalyseurs pour l'isomérisation d'alcools allyliques.
